# EUROPEAN PATENT APPLICATION

(11) **EP 2 292 205 A1**
(43) Date of publication of application: **09.03.2011**
(21) Application number: 09770251.8
(22) Date of filing: 26.06.2009
(51) Int. Cl.: A61H 33/14, A61H 33/10, A61H 35/00, A61K 33/00

(54) **PRESSURIZED GAS MIST BATHING SYSTEM**

(30) Priority: 27.06.2008 JP 2008168232
(71) Applicant: Nakamura, Shoichi, Higashichikuma-gun, Nagano 399-7502 (JP); ACP Japan Co. Ltd., Tokyo 113-0033 (JP)
(72) Inventor: NAKAMURA, Shoichi, Higashichikuma-gun Nagano 399-7502 (JP)
(74) Representative: Gill, David Alan
(86) International application number: PCT/JP2009/061721
(87) International publication number: WO 2009/157541

(57) **Abstract**

The invention is to provide a gas mist pressure bath device with a flat shaped patch, and is possible to efficiently absorb even gas of a small amount through a skin or mucous membrane of a human living-body, and can be made compact and reduce cost. The invention is concerned with the device for causing oxygen, carbon dioxide, or a mixed gas (called as "gas" hereafter) at density of not less than a predetermined value to contact the skin or mucous membrane of the living-body, and this device comprises a gas mist generating means 11 for generating and supplying a mist (called as "gas mist" hereafter) prepared by pulverizing and dissolving the gas and liquid, and a flat shaped living-body cover means 12 which is formed with a space of covering the living-body' s skin or mucous membrane and sealing inside thereof the gas mist from the gas mist generating means 11, and a pressurizing means 13 for pressurizing the inside of the living-body cover means 12 in order to cause the gas mist sealed within the living-body cover member at pressure more than the predetermined value to contact the living-body's skin or mucous membrane by means of the pressurizing means 13.

## Description

### Technical Field

The present invention relates gas mist pressure bath device, in which a gas mist is prepared by pulverizing and dissolving carbon dioxide, oxygen or a mixed gas of carbon dioxide and oxygen, and liquid, and the thus prepared gas mist is directly contacted to a skin or mucous membrane of a living-body for improving a blood circulation of the living-body.

### Background Art

It has conventionally been known that carbon dioxide (carbonic acid anhydride: CO₂, called as "carbon dioxide" hereafter) has both properties of being not only soluble in water (water-soluble) but also soluble in fat (fat-soluble) and if, therefore, only contacting the skin or mucous membrane of the living-body being as mixed with water and fat, carbon dioxide penetrates under a subcutaneous layer and expands blood vessels around penetrated carbon dioxide, and it works to improve a blood circulation. Owing to this action of accelerating the blood circulation, it displays various physiological effects such as dropping of blood pressure, improving of metabolism or accelerating to remove pain substance or waste product. Further, it has also anti-inflammation and anti-bacterial. Therefore, carbon dioxide has recently been given attention also from viewpoints of improving health or beauty other than the purpose of medical cares.

Carbon dioxide in the tissue of the living-body works to release oxygen carried in combination with hemoglobin in a red blood cell. Around parts at a high density of carbon dioxide, the red blood cell releases more oxygen. Thus, supply of oxygen to cells by the red blood cell is mainly controlled by carbon dioxide. In short, being without carbon dioxide, hemoglobin remains as combined with oxygen and the cell becomes unable to receive oxygen. As is seen, carbon dioxide is seen as a waste product resulted from action of oxygen, however, it plays in fact very important roles in the human living-body.

Further, in recent times, oxygen of high density has also widely been known as effective in activity of metabolism, fatigue recovery, stability of blood pressure and others.

As techniques of causing the living-body to absorb carbon dioxide at the comparatively narrow limited parts thereof, there have been disclosed as following.
(1) A device, which attaches a closing simple cover to the limited part of a human body and introduces carbon dioxide into the cover for carrying out the carbon dioxide bath (refer to, for example, Patent Document 1).
(2) A device, which inserts the limited part of the human body into the closing container (otherwise, attaching the container to the limited part of the human body) and introduces carbon dioxide into the container for carrying out the carbon dioxide bath (refer to, for example, Patent Document 2).
(3) A device, which attaches to the limited part of the human body a sealing and surrounding material composed of a container having an opening such as a bag body or a tubular body, seals an absorbing assistant material of helping skin-passing absorption of carbon dioxide in order to close the interior of the sealing and surrounding material, and introducing carbon dioxide thereinto for carrying out the carbon dioxide bath by (refer to, for example, Patent Document 3).

There have been proposed by present inventors the carbon dioxide pressure bath devices which are furnished with at least gas mist generating means, pressure means, and coating members of one or two layer structures for covering the skin or mucous membrane of the living-body and for causing carbon dioxide to contact the skin or mucous membrane at pressure more than a predetermined value.

### Citation List

### Patent Documents

Patent Document 1: Japanese Laid-Open Patent Publication No. 07-171189
Patent Document 2: Japanese Laid-Open Patent Publication No. 2007-252871
Patent Document 3: Domestic Re-publication WO2004/002393

### Summary of Invention

### Problems to be Solved by the Invention

However, in the devices for the prior art carbon dioxide bath described in the above mentioned patent documents 1 to 3, such problems were involved that the capacities of the container, cover or bag enclosing carbon dioxide were large, and if being wholly filled, a large quantity of carbon dioxide was consumed. But for putting the devices to practical use, it is inevitable to leave margins, to some extent, to the container, cover or bag in order to cope with users' various body figures. Making the enclosing space of carbon dioxide compact was difficult, and as a result, suppression of consuming amount of carbon dioxide was troublesome.

On the other hand, in the carbon dioxide pressure bath devices having already been proposed by the present inventors, it is possible to largely heighten an absorption rate of carbon dioxide into the skin or mucous membrane by causing carbon dioxide to contact the skin or mucous membrane at values higher than a predetermined pressure value by a pressure means, while a device like a compressor as the pressure means is necessary. Therefore, increase of cost is invited, while the device is large scaled, so that it is unsuitable to ordinary uses as in home. Further, the coating part has the complicated structure, so that a production cost rises.

Besides, there has not been present up to now a device which can absorb by skin-passing at efficiently not only carbon dioxide but oxygen and a mixed gas of carbon dioxide and oxygen.

In view of the above mentioned conventional problems, the invention employs a flat shaped patch, and is to provide a gas mist pressure bath device which is possible to efficiently absorb even the gas of a small amount through a skin or mucous membrane of the human living-body, and can be made compact and reduce cost.

### Means for Solving the Problem

For accomplishing the object, the invention is concerned with the device for causing oxygen, carbon dioxide, or a mixed gas (called as "gas" hereafter) at density of not less than a predetermined value to contact the skin or mucous membrane of the living-body, and this device is characterized by comprising a gas mist generating means for generating and supplying a mist (called as "gas mist" hereafter) prepared by pulverizing and dissolving the gas and liquid, and a flat shaped living-body cover means which is formed with a space of covering the living-body's skin or mucous membrane and sealing inside thereof the gas mist from the gas mist generating means in order to cause the gas mist sealed within the living-body cover member at a pressure more than the predetermined value to contact the living-body' s skin or mucous membrane. Herein, the instant gas mist pressure bath device is further provided with a pressurizing means for pressurizing the gas mist within the living-body cover member.

By the way, the invention refers it as "pulverizing and dissolving" to pulverize liquid into fine liquid drops, and cause to contact the gas (carbon dioxide, oxygen, or a mixed gas of carbon dioxide and oxygen).

With respect to the gas mist pressure bath device of the invention, concretely, the pressurizing means is composed of a ring member, and the flat shaped living-body cover means has a convex portion projecting outside of the living-body cover means and this ring member is passed at its center hole with the front end of the convex portion of the living-body cover member, and while taking out the gas mist from the end of the living-body cover member, the ring member is slid to the insertion side of the living body of the living-body cover member in order to reduce the capacity within the living-body cover member, thereby to carry out pressurization. At this time, in the living-body cover member, almost parallel ribs are preferably formed in a sliding direction of the ring member.

Otherwise, the pressurizing means is composed of a gas storage formed in the living-body cover means, projecting toward an outside of this living-body cover means, and pressurization is carried out by pressurizing this gas storage to exhaust carbon dioxide therein to the side of the skin or mucous membrane of the living body within the gas storage. At this time, a further covering member may be furnished to cover the gas storage.

On the other hand, suitably, the living-body cover means has, on its periphery, one or plural adhering means with viscosity to the skin or mucous membrane of the living body. In addition, it is desirable to furnish one or plural securing means for securing the living-body cover means to the living body.

In regard to the above mentioned liquid, suitable are water, ionic water, physiological salt solution, anti-allergic agent, anti-inflammatory agent, anti-febrile, anti-fungus agent, or anti-influenza virus. Otherwise, the above liquid is water containing one or plural medicines of menthol, vitamin E, vitamin C derivative, retinol, anesthetic, cyclodextrin, complex of photocatalyst and apatite, hyaluronic acid, coenzyme Q10, seed oil, propolith, or high density carbonate spring, ionic water, physiological salt solution, anti-allergic agent, anti-inflammatory agent, anti-febrile, anti-fungus agent, or anti-influenza virus.

Grain sizes of the gas mist supplied from the gas mist generating means to the living-body cover member are suitably not more than 10 µm.

In addition, optimum pressurization by the pressurizing means is 1.02 to 2.5 air pressure. Preferably, interval pressurization (pulse pressurization) is carried out by the pressurizing means of the living-body cover member.

The living-body cover means is suitably formed with any one or plural combination of a natural rubber, silicone rubber, polyethylene, polypropylene, polyvinylidene chloride, poly stylene, polyvinylacetate, polyvinylchloride, polyamide resin, polytetrafluoroethylene, woven fabric or non-woven fabric.

It is preferable that the living body cover means has a gas mist supply opening for introducing the gas mist supplied from the gas mist generating means into the living-body cover member, and this gas mist supply opening is provided therein with the check valve.

Desirably, the gas mist generating means has a gas mist supply pipe for supplying the gas mist into the living-body cover member, and this gas mist supply pipe has a filter for removing liquid drops attached to a pipe inside. Further, a whole or one of the gas mist supply pipe is suitably composed of a cornice shaped pipe, and this gas mist supply pipe is provided with the check valve.

### Advantageous Effect of the Invention

According to the gas mist pressure bath device of the invention, because a closed space for introducing gas is made a flat shaped patch, efficient skin-passing absorption is available in spite of gas of a small amount. In addition, with the flat shaped patch, it may be applied to various parts of the living body.

Being a very simple structure, anybody can easily use the present gas mist pressure bath device without necessitating a large scaled facility nor choosing places. Production costs can be reduced than hitherto.

### Brief Description of Drawings

[FIG. 1] A generally schematic view of the gas mist pressure bath device depending on a first embodiment of the invention;
[FIG. 2] A cross sectional typical view of the living body pressure bath patch depending on the first embodiment of the invention;
[FIG. 3] Typical views for explaining methods of using the gas mist pressure bath device depending on the first embodiment of the invention;
[FIG. 4] A generally schematic view of the gas mist pressure bath device depending on a second embodiment of the invention; [FIG. 5] A generally schematic view showing deformed view of the gas mist pressure bath device depending on the second embodiment of the invention;
[FIG. 6] Typical views showing the shape examples of the adhesive parts of the patches for the living body in the gas mist pressure bath device depending on the second embodiment of the invention; and
[FIG. 7] A typical view showing one example of the gas mist supply pipe used to the gas mist pressure bath device depending on the invention.

### Description of Embodiments

In the following description, explanations will be made to embodiments of this invention, referring to the attached drawings.

### [First Embodiment]

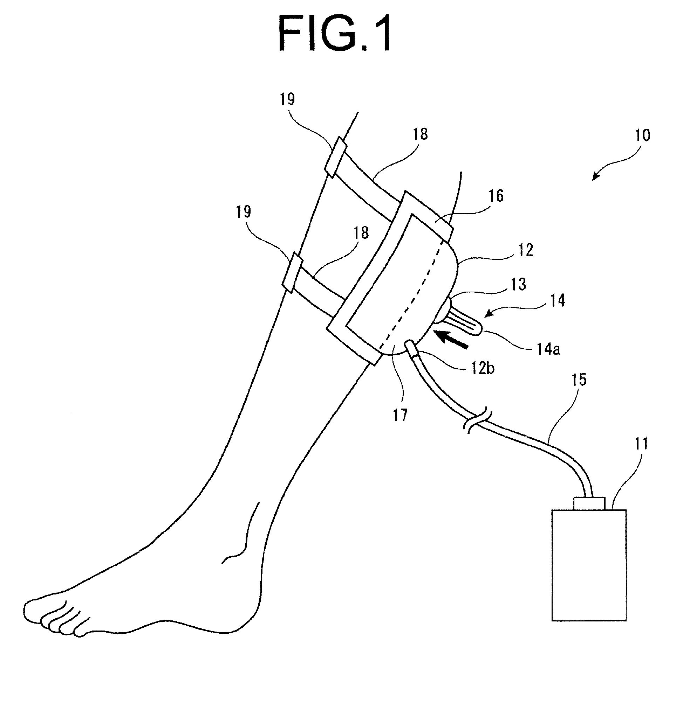

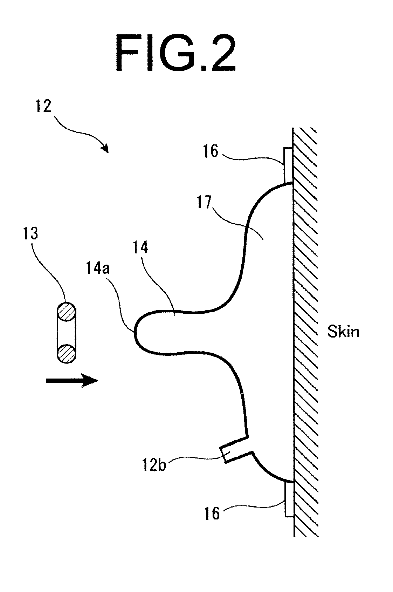

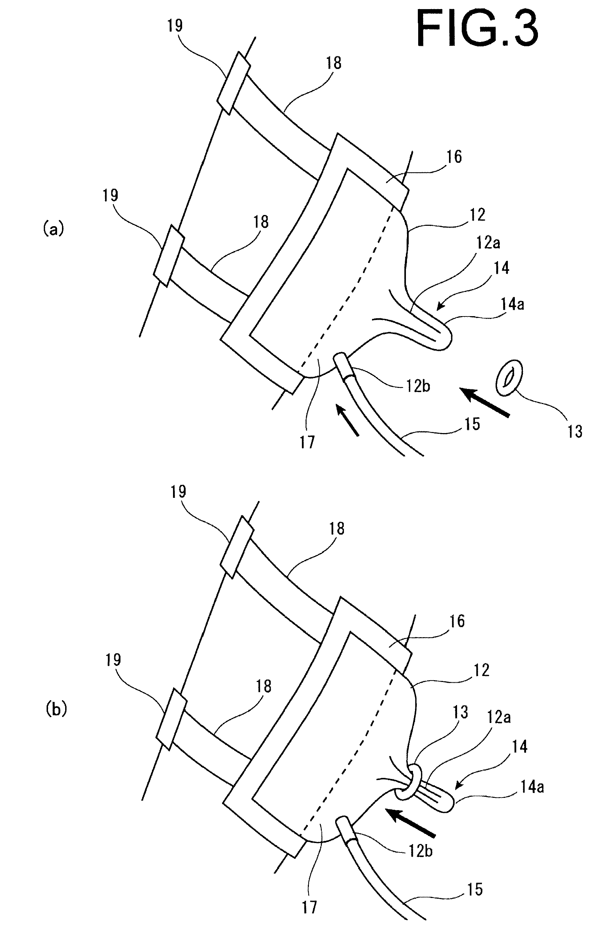

FIG. 1 is the generally schematic view of the gas mist pressure bath device depending on the first embodiment of the invention, FIG. 2 is the cross sectional typical view of the living body pressure bath patch in the gas mist pressure bath device, and FIG. 3 is the typical views for explaining the methods of using the gas mist pressure bath device.

The gas mist pressure bath device 10 of this embodiment is, as shown in these FIG. s, composed of the gas mist generating device 11 for generating and supplying the gas mist, the flat shaped living-body pressure bath patch 12, and a reducing ring 13 for pressurizing the gas mist sealed in the living-body pressure bath patch 12, this living-body pressure bath patch 12 covering the living body's limited part (herein, as an example, leg of the living body), forming a space for storing the gas mist and causing the skin or mucous membrane of the living body to directly contact the gas mist and absorb it.

The gas mist generating device 11 has inside a liquid supply means and a gas supply means such as a gas bomb, otherwise, this is connected to an outside liquid supply means and the gas supply means such as the gas bomb, and generates a mist (gas mist) of having pulverized and liquefied the liquid and gas, and supplies into the living-body pressure patch 12. For example, other than a device which is furnished with a fluid nozzle so that high flowing speed of gas from the gas supply means is utilized to pulverize and dissolve the liquid for generating the gas mist, there may be used various kinds of gas mist generating devices, for example, devices of generating the gas mist by jetting gas into the liquid at high pressure. Optimum grain sizes of the mist generated are not more than 10 µm.

In regard to the liquid to be used for generating the gas mist, other than water, ion water or physiological salt solution, it is preferable to use medical liquids useful to users' diseases, symptoms or other conditions, such as anti-allergic agent, anti-inflammatory agent, anti-analgesic and febrile agent, anti-fungus agent, or anti-influenza virus. This liquid is further possible to generate a synergistic effect with a gas physiological action by coupling with single or plurality of menthol having a cooling action; vitamin E accelerating circulation of the blood; vitamin C derivative easily to be absorbed to a skin tissue and having a skin beautifying effect; retinol normalizing a skin heratinizing action and protecting the mucous membrane; anesthetic moderating irritation to the mucous membrane; cyclodextrin removing odor; a complex of photocatalysis and apatite having disinfection and anti-phlogistic; hyaluronic acid having excellent water holding capacity and a skin moisture retention effect; coenzyme Q10 activating cells and heightening immunization; a seed oil containing anti-oxidation and much nutrient; or propolith having anti-oxidation, anti-fungus, anti-inflummatory agent, pain-killing, anesthetic, and immunity. Further, high density carbonate spring agent having main components of carbonate and organic acid (as one example of active ingredients, sulfate, carbonate, organic acid or sodium dichloroisocyanurate) may be added.

The generated gas mist is supplied into the living-body pressure bath patch 12 through the gas mist supply pipe 15 connected to the gas mist generating device 11. Inside of the gas mist supply pipe 15, a check valve is provided for checking back flows of the gas mist. In addition, the gas mist supply pipe 15 has a liquid drop-removing filter (not shown) for removing excessive liquid drops attached to the inside of the pipe.

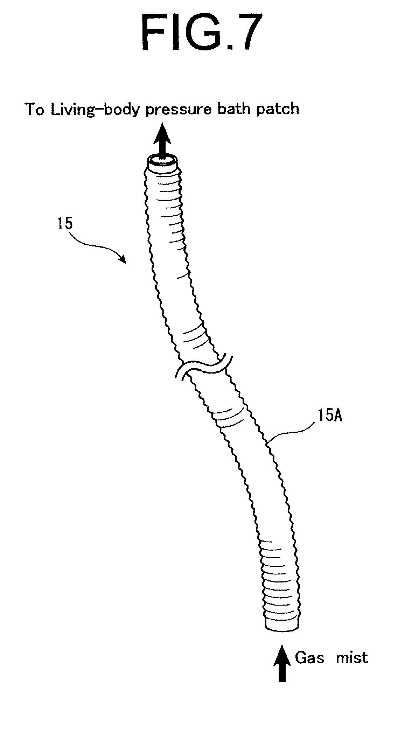

Further, as shown in FIG. 7, preferably, the gas mist supply pipe 15 is overall or partially composed of a soft cornice shaped pipe 15A of a large diameter. If composing with such a corniced pipe 15A, the gas mist supply pipe 15 is freely bent and may be expanded so that the user is not restricted in action. Even if the gas mist flowing in the gas mist supply pipe 15 becomes gradually liquefied, the liquid can be removed through concaves and convexes of the cornice.

The living-body pressure bath patch 12 is a flat shaped patch for covering the skin or mucous membrane of the living body. Inside, a space (gas mist storage 17) is provided for sealing the gas mist, while at an almost central part, a convex portion 14 is formed toward the outside of the patch. A raw material of the living-body pressure bath patch 12 is made of the woven or non-woven fabric of a non-air permeable and non-moisture permeable material, for example, preferably, the natural rubber, silicone rubber, polyethylene, polypropylene, polyvinylidene, polystylene, polyvinylacetate, polyvinyl chloride, polyamide resin, polytetrafluoroethylene.

Further, the living-body pressure bath patch 12 has a structure adhered and fixed to the limited part of the living body (herein, as the example, a leg of the living body). In particular, for sealing the inside of the living-body pressure bath patch 12 and avoiding leakage of the gas mist, it has an adhesive part (adhering means) 16 on the marginal portion thereof. At the side of the adhesive part 16 contacting the skin or mucous membrane of the living body, a material is arranged for contacting the skin or mucous membrane of the living body. The adhesive material is preferably, for example, a viscoelastic gel of polyurethane or silicone rubber. Further, this material is optimum which is exchangeable each time when viscosity becomes weak.

The living-body pressure bath patch 12 has a stopping belt 18 and a belt stopper 19 as a securing means for exactly fixing to the living body. The stopping belt 18 is suitably made of such materials having stretching property as a rubber for applying to users' body figures or requested parts. Figure shows an example of arranging four pieces of stopping belts 18 and securing at two upper and lower parts, and in view of the figures of the living-body pressure bath patch 12, the number and places are adjusted. The belt stopper 19 is desirably one-touch buckle for easily attaching and detaching the stopping belt 18. An adjuster may be provided for adjusting a belt length of the stopping belt 18.

At the convex portion 14 of the living-body pressure bath patch 12, it is preferable that ribs 12a are formed in vicinity of the front end 14a thereof almost in parallel to a sliding direction of the reducing ring 13 for easily tightening and sliding the reducing ring 13. By the way, the linear ribs 12a are illustrated, but curved shapes are also enough. The living- body pressure bath patch 12 is furnished with a gas mist supply mouth 12b for introducing the gas mist supplied from the gas mist generating device 11 into the living-body pressure bath patch 12. The gas mist supply mouth 12b is formed inside with a check valve for checking back flow of the gas mist.

The reducing ring 13 is the pressurizing means for passing and sliding the convex front end 14a through its central hole and pressurizes (around 1.02 to 2.5 air pressure) the inside of the living-body pressure bath patch 12 while extracting gas mist having gathered at the convex front end 14a to the side of the user' s skin or mucous membrane. Therefore, the reducing ring 13 is preferably made of an elastic member (for example, rubber or the like), because when attaching to the living-body pressure bath patch 12, the reducing ring 13 extends for passing the convex front end 14a through its central hole, and after then reduces for extracting the gas mist gathering at the convex front end 14a.

Referring to FIG. 3, explanation will be concretely made to the method of the gas mist pressure bath using the gas mist pressure bath device. At first, as shown FIG. 3(a), the living-body pressure bath patch 12 is attached to a user's requested portion by the adhesive part 16 and is made sealed inside, and at this time, air within the living-body pressure bath patch 12 is extracted as possible. In addition, the living-body pressure bath patch 12 is secured to the user's body with the stopping belt 18 and the belt stopper 19. Next, the gas mist supply pipe 15 is connected to the gas mist supply mouth 12b of the living-body pressure bath patch 12, and the gas mist is supplied from the gas mist generating device 11 into he living-body pressure bath patch 12. Then, it is not necessary to fill the gas mist in the whole of the living-body pressure bath patch 12. When storing the gas mist of necessary minimum within the living-body pressure bath patch 12, the gas mist supplied from the gas mist generating device 11 is stopped.

As shown in FIG. 3(c), the convex front end 14a of the living-body pressure bath patch 12 is passed in the central hole of the reducing ring, the living-body pressure bath patch 12 is squeezed at the convex front end 14a, and while extracting the gas mist at the side of the convex front end 14a through the central hole of the reducing ring 13, the reducing ring 13 is slid toward the skin or mucous membrane of the user. Thereby, the capacity of the gas mist storage 17 becomes gradually smaller and the interior of the living-body pressure bath patch 12 is pressurized. The reducing ring 13 is slid until a moderately pressurizing condition (around 1.02 to 2.5 air pressure), and fixedly stopped there. Holding this condition, the gas mist pressure bath is carried out with respect to the portion attached with the living-body pressure bath patch 12. Thus, by keeping the moderately pressurizing condition within the living-body pressure bath patch 12, the gas mist filled in the living-body pressure bath patch 12 can be efficiently absorbed into the skin or mucous membrane.

By the way, the present embodiment can also employ a clip instead of the reducing ring. In short, if it is possible that the convex front end of the living-body pressure bath patch is kept as a condition of extracting the gas mist and the moderately pressurizing condition is held at the gas mist storage, any means is available. In this embodiment, the reducing ring is manually slid and fixed, and such an embodiment may be allowed which carries out such performance mechanically by, for example, a driving device. In addition, since pressurization in the gas mist pressure bath heightens the effects by pressurizing in pulsing at predetermined interval, the reducing ring may be slid intermittently at fixed rhythm. As to the pressurizing interval at such a case, if synchronizing with pulsations, the effects are more heightened.

### [Second Embodiment]

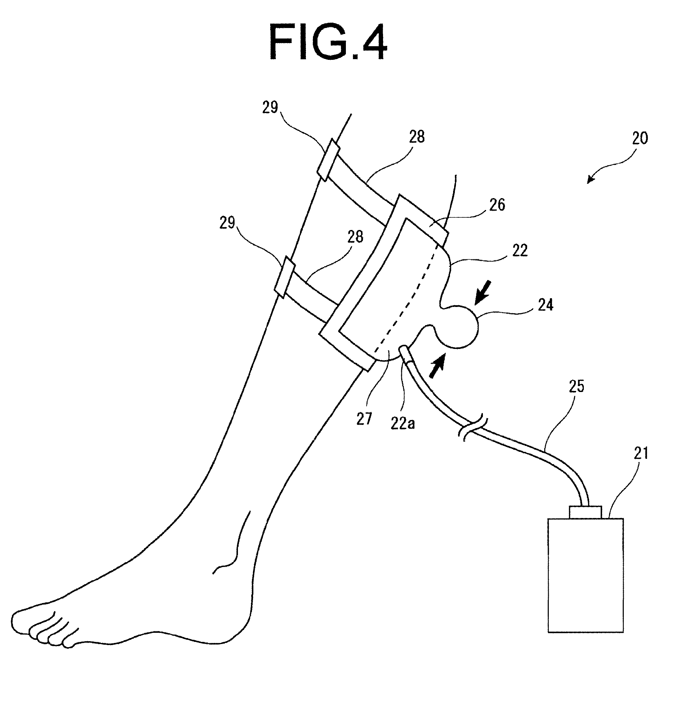

FIG. 4 is the generally schematic view of the gas mist pressure bath device depending on the second embodiment of the invention. The gas mist pressure bath device of this embodiment has the same structure of the first embodiment other than the structure in relation with the shape of the living-body pressure bath patch, and explanation thereof will be omitted in the following.

The gas mist pressure bath device 20 of the present embodiment is, as shown in FIG. 4, composed of the gas mist generating device 21 of generating and supplying the gas mist and the living-body pressure bath patch 22 of covering the limited part of the living body (herein, as the example, the human body's leg) as well as forming a space for storing the gas mist, and causing the skin or mucous membrane of this requested living body part to directly contact and absorb the gas mist. The living-body pressure bath patch 22 is formed, almost at the center, with a spherical pressurizing part (gas storage) 24 opposite to the outside of the patch and a flat shape space (gas mist collecting part 27) in an inside thereof.

For practicing the gas mist pressure bath, the living- body pressure bath patch 22 is adhered by the adhesive part 26 to the user's requested position, and the inside of the living- body pressure bath patch 22 is made almost sealed. Then, adhesion is done under the condition that air is extracted as possible from the inside of the living-body pressure bath patch 22. Further, the living-body pressure bath patch 22 is secured with a stopping belt 28 and a belt stopper 29. Subsequently, the gas mist supply pipe 25 is connected to the gas mist supply mouth 22a of the living- body pressure bath patch 22, and the gas mist is supplied from the gas mist generating device 21 into the living-body pressure bath patch 22. When the gas mist gathers as desired in the living-body pressure bath patch 22, the gas mist supplied from the gas mist generating device 11 is stopped. The gas mist in the pressurizing part 24 is exhausted to the side of the gas mist storage 27 by pressurizing as crushing the pressurizing part 24 of the living-body pressure bath patch 22 to make the inside of the living-body pressure bath cover 22 moderate (around 1.02 to 2.5 air pressure). Thereby, the gas mist filled in the living-body pressure bath patch 22 can be efficiently absorbed into the skin or mucous membrane.

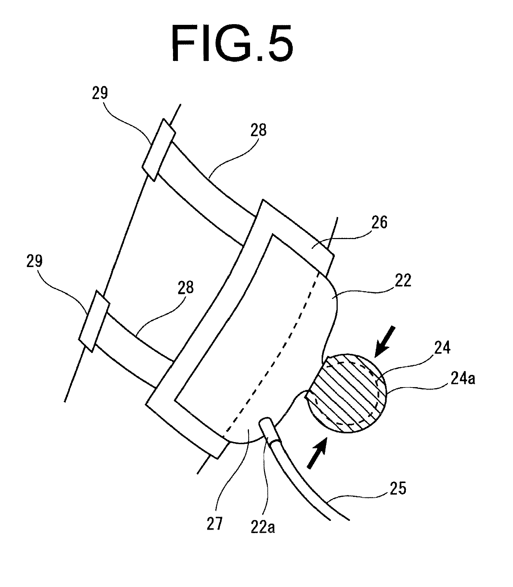

By the way, the pressurizing part 24 may be provided with an exclusively used cover. FIG. 5 is the generally schematic view showing the deformed example of the gas mist pressure bath device depending on the second embodiment of the invention. As shown in FIG. 5, the pressurizing part 24 is covered with a spherical cover 24a having flexibility and elasticity. With such a structure, the pressurizing part 24 can increase its strength and using feeling. This pressurizing part cover 24a may have such a structure enabling to remove the pressurizing part cover 24a only or together with the pressurizing part 24 from the living-body pressure bath patch 22.

The above mentioned embodiment has the structure manually pressurizing the pressurizing part 24, but may carry out it mechanically with a driving device. Further, since pressurization in the gas mist bath heightens effect by doing in pulse at predetermined interval, the pressurizing part 24 may be pressurized intermittently at constant rhythm. Pressurizing interval then heightens effects by synchronizing with stroke of pulsation.

The above explanation has been made to the embodiments of the invention, and in the first and second practiced embodiments, the structures have the gas mist supply mouth for supplying the gas mist to the living-body pressure bath patch, but instead, the invention may have such an embodiment where the gas mist supply pipe is connected at its one end to the gas mist generating device and is inserted at the other end into the living-body pressure bath patch. In such a case, there is provided, inside of the gas mist supply pipe, the check valve for checking back flow of the gas mist.

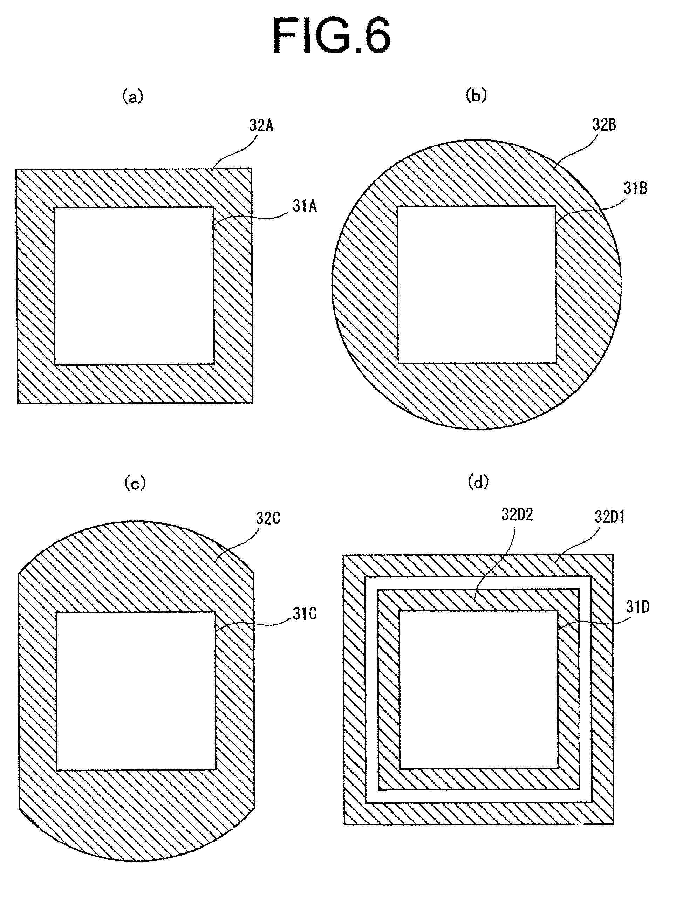

The drawings concerning the above mentioned embodiments of the living-body pressure bath patch is rectangular, but the invention does not limit thereto, and meeting the adhering parts of the living body to be attached, various shapes of patches are available (for example, a rectangular shape without one of vertical lines, for shoulder or back). In addition, the adhesive parts may be assumed to be various. FIG. 6 shows examples of the adhesive parts. As shown in FIG. 6(a), margins of the living-body pressure bath patch 31A are similar to those of the adhesive part 32A, and as shown in FIG. 6(b) and (c), the shapes of the perimeters of the living-body pressure bath patches 31B and 31C may be quite different from those of the adhesive parts 32B and 32Ct. Further, as seeing in FIG. 6(d), encircling the living-body pressure bath patch 31D, plural (herein, as the example, two) adhesive parts 32D1, 32D2 may be provided.

Further, in the above embodiments, the securing means of the living-body pressure bath patch is composed of the stopping belt and the belt stopper, but for example, the string may be tied fixedly at the parts merely attached by the string, or a rubber belt and a face fastener are combined and fixed. Apart from the living-body pressure bath patch, a supporter is prepared (an opening is provided for exposing the convex part of the living-body pressure bath patch and the gas mist supply mouth) having the shapes (for example, waist, shoulders and others) meeting the parts to be attached of the living body, and covering on the attached living-body pressure bath patch. In sum, any structures are sufficient, if being able to exactly fasten the living-body pressure bath patch.

Omitting illustrations in the above embodiments, the living-body pressure bath patch may be inserted inside with gas density measuring instrument (carbon dioxide density measuring instrument and oxygen density measuring instrument) or pressure gauge.

Being structured as above mentioned, according to the living-body pressure bath patch of the invention, the closed space for introducing the gas mist is prepared inside of the flat shaped patch, whereby even the gas of the small amount is able to be efficiently absorbed via the skin-pass. If the patch is flat- shaped, it can be applied to various parts of the living body.

Further, being very simple structure, any one can easily use without requesting large scaled equipment or selecting operating places. Comparing with the prior art, a production cost can be reduced.

The above explanation has been made to the embodiments of the invention, but the invention is not limited to such embodiments, and so far as not deviating from the subject matter of the invention, various kinds of embodiments are, of course, available.

### Industrial Applicability

Thus, the present invention relates to the gas mist pressure bath device, in which the gas mist is prepared by pulverizing and dissolving carbon dioxide and oxygen or the mixed gas of carbon dioxide and oxygen, and the gas mist is directly contacted to the skin or mucous membrane of the living-body for improving the blood circulation of the living-body, and has an industrial applicability.

### Description of Symbols

- 10, 20:: Gas mist pressure bath device
- 11, 21:: Gas mist generating device
- 12, 22, 31A, 31B, 31C, 31D:: Living-body pressure bath patch
- 12a:: Rib
- 12b, 22a:: Gas mist supply mouth
- 13:: Reducing ring
- 14:: Convex portion
- 14a:: Front end of convex portion
- 15, 25:: Gas mist supply pipe
- 15A:: Cornice shaped pipe
- 16, 26, 32A, 32B, 32C, 32D1, 32D2:: Adhesive part
- 17, 27:: Gas mist storage
- 18, 28:: Stopping belt
- 19, 29:: Belt stopper
- 24:: Pressurizing part (gas storage)
- 24a:: Cover for pressurizing part

## Claims

1. A gas mist pressure bath device for causing oxygen, carbon dioxide or a mixed gas (called as "gas" hereafter) of oxygen and carbon dioxide at density of not less than a predetermined value to contact a skin or mucous membrane of a living-body, comprising:
a gas mist generating means for generating and supplying a mist (called as "gas mist" hereafter) prepared by pulverizing and dissolving the gas and liquid, and
a flat shaped living-body cover member for covering the living-body's skin or mucous membrane and forming a space for sealing inside thereof the gas mist from the gas mist generating means,
wherein the gas mist sealed within the living-body cover means is caused to contact the living-body's skin or mucous membrane at a pressure more than a predetermined value.

2. A gas mist pressure bath device as set forth in claim 1, wherein there is further provided a pressurizing means for pressurizing the gas mist within the living-body cover means.

3. A gas mist pressure bath device as set forth in claim 2, wherein the pressurizing means is composed of a ring member,
the flat shaped living-body cover means has a convex portion projecting toward the outside of the living-body cover means,
the ring member is passed at its center hole with the front end of the convex portion of the living-body cover means, and while taking out the gas mist from the front end, the ring member is slid to the side of the skin or mucous or membrane of the living body in order to reduce the capacity within the living-body cover means, thereby to carry out pressurization.

4. A gas mist pressure bath device as set forth in claim 3, wherein, in the living-body cover means, almost parallel ribs are formed in a sliding direction of the ring member.

5. A gas mist pressure bath device as set forth in claim 2, wherein the pressurizing means is composed of a gas storage formed in the living-body cover member, projecting toward the outside of the living-body cover means, and
the gas storage is pressurized to exhaust the gas mist therein to the side of the skin or mucous membrane of the living-body to carry out pressurization.

6. A gas mist pressure bath device as set forth in claim 5, wherein there is further provided a covering member may be furnished to cover the gas storage.

7. A gas mist pressure bath device as set forth in claim 1, wherein the living-body cover means has, on its perimeter, one or plural adhesive means having adhesion to the skin or mucous membrane of the living body

8. A gas mist pressure bath device as set forth in claim 7, wherein there are furnished one or plural securing means for securing the living-body cover means to the living body.

9. A gas mist pressure bath device as set forth in claim 1, wherein the above mentioned liquid is used as any one or plural combination of water, ionic water, physiological salt solution, anti-allergic agent, anti-inflammatory agent, anti-febrile, anti-fungus agent, or anti-influenza virus.

10. A gas mist pressure bath device as set forth in claim 1, wherein the above mentioned liquid is any of water, ionic water, physiological salt solution, anti-allergic agent, anti-inflammatory agent, anti-febrile, anti-fungus agent, or anti-influenza virus, which are contained with one or plural medicines of menthol, vitamin E, vitamin C derivative, retinol, anesthetic, cyclodextrin, complex of photocatalyst and apatite, hyaluronic acid, coenzyme Q10, seed oil, propolith, and high density carbonate spring.

11. A gas mist pressure bath device as set forth in claim 1, wherein grain sizes of the gas mist supplied from the gas mist generating means to the living-body cover member are suitably not more than 10 µm.

12. A gas mist pressure bath device as set forth in claim 2, wherein pressurization by the pressurizing means is 1.02 to 2.5 air pressure.

13. A gas mist pressure bath device as set forth in any one of claims 2 to 5, wherein interval pressurization is carried out by the pressurizing means.

14. A gas mist pressure bath device as set forth in claim 1, wherein the living-body cover means is suitably formed with any one or plural combination of a natural rubber, silicone rubber, polyethylene, polypropylene, polyvinylidene chloride, poly stylene, polyvinylacetate, polyvinylchloride, polyamide resin, polytetrafluoroethylene, woven fabric or non-woven fabric.

15. A gas mist pressure bath device as set forth in claim 1, wherein the living-body cover means has a gas mist supply mouth for introducing the gas mist supplied from the gas mist generating means into the living-body cover means, and
the gas mist supply mouth is provided inside with a check valve.

16. A gas mist pressure bath device as set forth in claim 1, wherein the gas mist generating means has a gas mist supply pipe for supplying the gas mist into the living-body cover means, and
the gas mist supply pipe has a filter for removing liquid drops attached to the inside of the pipe.

17. A gas mist pressure bath device as set forth in claim 1, wherein the gas mist generating means has a gas mist supply pipe for supplying the gas mist into the living-body cover means, and
the whole or one part of the gas mist generating means are composed of cornice shaped pipes.

18. A gas mist pressure bath device as set forth in claim 1, wherein the gas mist generating means has the gas mist supply pipe for supplying the gas mist into the living-body cover means, and
the gas mist supply mouth is provided with a check valve.
